# EUROPEAN PATENT APPLICATION

(11) **EP 4 767 948 A1**
(43) Date of publication of application: **01.07.2026**
(21) Application number: 24912365.4
(22) Date of filing: 11.12.2024
(51) Int. Cl.: A61B 6/51, A61B 6/03, A61C 19/04

(54) **DENTAL TREATMENT ASSISTANCE SYSTEM**

(30) Priority: 25.12.2023 JP 2023218425
(71) Applicant: Watahiki, Junichi, Tokyo 103-0022 (JP); Dental Brain Inc., Tokyo 100-0004 (JP)
(72) Inventor: WATAHIKI Junichi, Tokyo 103-0022 (JP)
(74) Representative: Cyrson, Matthew Dominic
(86) International application number: PCT/JP2024/043732
(87) International publication number: WO 2025/142476

(57) **Abstract**

[Problem] To provide a novel technology that can be used to assist treatment such as orthodontic treatment. [Solution] This dental treatment assistance system comprises: a measurement point information acquisition unit that acquires measurement point information indicating a measurement point for orthodontic diagnosis on the basis of an image obtainable by X-ray imaging of the head of a subject, a magnetic resonance image of the head of the subject, or an image obtainable by computer tomographic imaging of the head of the subject; a position information acquisition unit that, on the basis of the head measurement point information, acquires position information during imaging indicating the positions of a front tooth, a molar or the jawbone of the subject during imaging; a planned position information acquisition unit that acquires planned position information indicating the position when it is assumed that the tooth or the jawbone is moved; and a movement information generation unit that, on the basis of the measurement point information, the position information during imaging, and the planned position information, generates movement information indicating a movement amount from the position before movement when it is assumed that the front tooth, the molar, or the jawbone is moved.

## Description

### Technical Field:

The present invention relates to support for dental treatment.

### Background Art:

Conventionally, orthodontic treatment has been performed for the purpose of aligning teeth and occlusion and obtaining proper occlusion (normal occlusion). In orthodontic treatment, orthodontic appliances are used to apply force to teeth gradually to move the teeth, and in some cases, the maxillary and mandibular bones are surgically moved, thereby performing treatment aimed at obtaining esthetic and healthy tooth alignment and occlusion.

Accordingly, an orthodontic treatment plan, in which it is examined in advance whether teeth and/or the maxillary and mandibular bones should be moved and how they should be moved, is important. In formulating an orthodontic treatment plan, for example, cephalometric analysis is widely performed, and devices for supporting such analysis have also been proposed (for example, Patent Document 1). In cephalometric analysis, measurement points (landmarks) are set on a standardized head X-ray photograph of a subject requiring orthodontic treatment. Next, based on the set measurement points, angles formed between various planes or axes and reference planes are measured, and analysis is performed by comparing the obtained angles with average values of individuals having normal occlusion, thereby clarifying anatomical characteristics of individuals with malocclusion and enabling extraction of problems. In addition, cephalometric analysis is sometimes used for clinical and research purposes as an evaluation of treatment during and after orthodontic treatment.

### Prior Arts Documents: Patent Documents:

Patent Document 1: Japanese National Publication of International Patent Application No. 2020-534037

### Summary of the Invention:

### Problem to be Solved by the Invention

An object of the present invention is to provide a novel technology that can be used to support dental treatment.

### Means for Solving the Problem

As described above, treatment planning using cephalometric analysis has been performed. In conventional cephalometric analysis methods, although such methods are used to extract problems of a subject (such as a patient) having malocclusion, it has been difficult to set specific objectives of a treatment plan. In addition, several methods for setting treatment objectives have been reported; however, such methods deal with only numerical targets or drawings reflecting the concept of a particular individual practitioner, and thus have significant problems in terms of accuracy and reproducibility, and therefore have not been widely adopted. Accordingly, as a result of intensive research, the inventor has found a novel method that enables treatment planning to be formulated more easily, and have completed the present invention.

The present invention, for example, displays positions of upper and lower anterior teeth and molars as numerical data and image data by using a plurality of reference planes defined based on anatomical structures of the maxillary and mandibular bones and the skull. According to the present invention, it becomes possible to formulate an orthodontic treatment plan that takes harmony of the maxillofacial region into consideration. Specifically, the shape of the subject's jaws and characteristics of the skull can be grasped more accurately, and a treatment plan can be established based thereon. The present invention can provide a more effective and efficient approach in orthodontic treatment.

In addition, the present invention is applicable not only to orthodontic treatment but also to a wide range of dental treatments such as prosthodontic treatment.

The aspects of the present invention are as follows.
[1] A dental treatment support system comprising:
   a measurement point information acquisition unit configured to acquire measurement point information indicating measurement points corresponding to parts of a head, based on an image obtained by imaging a subject's head X-ray, a magnetic resonance image of the subject's head, or an image obtained by computed tomography of the subject's head;
   a position information acquisition unit configured to acquire, based on the measurement point information, imaging-time position information indicating positions of anterior teeth, molars, or jaw bones of the subject at the time of imaging;
   a planned position information acquisition unit configured to acquire planned position information indicating positions when the anterior teeth, molars, or jaw bones are assumed to be moved; and
   a movement information generation unit configured to generate movement information indicating amounts of movement from positions of the anterior teeth, molars, or jaw bones before movement to positions when the anterior teeth, molars, or jaw bones are assumed to be moved, based on the measurement point information, the imaging-time position information, and the planned position information.
[2] The dental treatment support system according to [1], wherein the movement information includes at least one of:
   information indicating an inclination movement amount of anterior teeth or molars;
   information indicating an intrusion amount or an extrusion amount of anterior teeth or molars;
   information indicating a translational movement amount of anterior teeth or molars;
   information indicating an inclination movement amount of jaw bones;
   information indicating a translational movement amount of jaw bones; and
   information indicating an intrusion amount or an extrusion amount of jaw bones.
[3] The dental treatment support system according to [2], wherein the movement information generation unit generates the information indicating the inclination movement amount of anterior teeth or molars as a change amount in an angle of a tooth axis relative to a reference plane serving as a reference for inclination.
[4] The dental treatment support system according to [2], wherein the movement information generation unit generates the information indicating the intrusion amount or the extrusion amount of anterior teeth or molars as a length between a position of a tooth before movement and a position of the tooth when assumed to be moved, in a direction perpendicular to a reference plane serving as a reference for movement.
[5] The dental treatment support system according to [2], wherein the movement information generation unit generates the information indicating the translational movement amount of anterior teeth or molars as a length between a position of a tooth before movement and a position of the tooth when assumed to be moved, in a direction along a reference plane serving as a reference for movement.
[6] The dental treatment support system according to [2], wherein the movement information generation unit generates the information indicating the inclination movement amount of jaw bones as a change amount in an angle between a reference plane serving as a reference for inclination and a plane that is at least in contact with the jaw bone.
[7] The dental treatment support system according to [2], wherein the movement information generation unit generates the information indicating the intrusion amount or the extrusion amount of jaw bones as a length between a position of a jaw bone before movement and a position of the jaw bone when assumed to be moved, in a direction perpendicular to a reference plane serving as a reference for movement.
[8] The dental treatment support system according to [2], wherein the movement information generation unit generates the information indicating the translational movement amount of jaw bones as a length between a position of a jaw bone before movement and a position of the jaw bone when assumed to be moved, in a direction along a reference plane serving as a reference for movement.
[9] The dental treatment support system according to any one of [1] to [8], further comprising a position identification support unit configured to generate position information generation support information for supporting identification of positions of anterior teeth, molars, or jaw bones of the subject by using the measurement point information acquired by the measurement point information acquisition unit,
   wherein the position information acquisition unit acquires the imaging-time position information based on user input using the position information generation support information generated by the position identification support unit.
[10] The dental treatment support system according to any one of [1] to [8],
   wherein the measurement point information acquisition unit acquires the measurement point information based on a standardized head X-ray photograph, which is an image obtained by imaging a subject's head X-ray.
[11] An information processing method executed by a computer, comprising:
   acquiring measurement point information indicating measurement points corresponding to parts of a head, based on an image obtained by imaging a subject's head X-ray, a magnetic resonance image of the subject's head, or an image obtained by computed tomography of the subject's head;
   acquiring, based on the measurement point information, imaging-time position information indicating positions of anterior teeth, molars, or jaw bones of the subject at the time of imaging;
   acquiring planned position information indicating positions when the anterior teeth, molars, or jaw bones are assumed to be moved; and
   generating movement information indicating amounts of movement from positions of the anterior teeth, molars, or jaw bones before movement to positions when the anterior teeth, molars, or jaw bones are assumed to be moved, based on the measurement point information, the imaging-time position information, and the planned position information.
[12] The method according to [11],
   wherein the movement information includes at least one of:
   information indicating an inclination movement amount of anterior teeth or molars;
   information indicating an intrusion amount or an extrusion amount of anterior teeth or molars;
   information indicating a translational movement amount of anterior teeth or molars;
   information indicating an inclination movement amount of jaw bones;
   information indicating a translational movement amount of jaw bones; and
   information indicating an intrusion amount or an extrusion amount of jaw bones.
[13] The method according to [12],
   wherein the information indicating the inclination movement amount of anterior teeth or molars is generated as a change amount in an angle of a tooth axis relative to a reference plane serving as a reference for inclination.
[14] The method according to [12],
   wherein the information indicating the intrusion amount or the extrusion amount of anterior teeth or molars is generated as a length between a position of a tooth before movement and a position of the tooth when assumed to be moved, in a direction perpendicular to a reference plane serving as a reference for movement.
[15] The method according to [12],
   wherein the information indicating the translational movement amount of anterior teeth or molars is generated as a length between a position of a tooth before movement and a position of the tooth when assumed to be moved, in a direction along a reference plane serving as a reference for movement.
[16] The method according to [12],
   wherein the information indicating the inclination movement amount of jaw bones is generated as a change amount in an angle between a reference plane serving as a reference for inclination and a plane that is at least in contact with the jaw bone.
[17] The method according to [12],
   wherein the information indicating the intrusion amount or the extrusion amount of jaw bones is generated as a length between a position of a jaw bone before movement and a position of the jaw bone when assumed to be moved, in a direction perpendicular to a reference plane serving as a reference for movement.
[18] The method according to [12],
   wherein the information indicating the translational movement amount of jaw bones is generated as a length between a position of a jaw bone before movement and a position of the jaw bone when assumed to be moved, in a direction along a reference plane serving as a reference for movement.
[19] The method according to any one of [11] to [18], further comprising:
   generating position information generation support information for supporting identification of positions of anterior teeth, molars, or jaw bones of the subject by using the measurement point information,
   wherein the imaging-time position information is acquired based on user input using the position information generation support information.
[20] The method according to any one of [11] to [18],
   wherein the measurement point information is acquired based on a standardized head X-ray photograph, which is an image obtained by imaging a subject's head X-ray.
[21] A program for causing a computer to function as:
   a measurement point information acquisition unit configured to acquire measurement point information indicating measurement points corresponding to parts of a head, based on an image obtained by imaging a subject's head X-ray, a magnetic resonance image of the subject's head, or an image obtained by computed tomography of the subject's head;
   a position information acquisition unit configured to acquire, based on the measurement point information, imaging-time position information indicating positions of anterior teeth, molars, or jaw bones of the subject at the time of imaging;
   a planned position information acquisition unit configured to acquire planned position information indicating positions when the anterior teeth, molars, or jaw bones are assumed to be moved; and
   a movement information generation unit configured to generate movement information indicating amounts of movement from positions of the anterior teeth, molars, or jaw bones before movement to positions when the anterior teeth, molars, or jaw bones are assumed to be moved, based on the measurement point information, the imaging-time position information, and the planned position information.
[22] The program according to [21],
   wherein the movement information includes at least one of:
   information indicating an inclination movement amount of anterior teeth or molars;
   information indicating an intrusion amount or an extrusion amount of anterior teeth or molars;
   information indicating a translational movement amount of anterior teeth or molars;
   information indicating an inclination movement amount of jaw bones;
   information indicating a translational movement amount of jaw bones; and
   information indicating an intrusion amount or an extrusion amount of jaw bones.
[23] The program according to [22],
   wherein the movement information generation unit generates the information indicating the inclination movement amount of anterior teeth or molars as a change amount in an angle of a tooth axis relative to a reference plane serving as a reference for inclination.
[24] The program according to [22],
   wherein the movement information generation unit generates the information indicating the intrusion amount or the extrusion amount of anterior teeth or molars as a length between a position of a tooth before movement and a position of the tooth when assumed to be moved, in a direction perpendicular to a reference plane serving as a reference for movement.
[25] The program according to [22],
   wherein the movement information generation unit generates the information indicating the translational movement amount of anterior teeth or molars as a length between a position of a tooth before movement and a position of the tooth when assumed to be moved, in a direction along a reference plane serving as a reference for movement.
[26] The program according to [22],
   wherein the movement information generation unit generates the information indicating the inclination movement amount of jaw bones as a change amount in an angle between a reference plane serving as a reference for inclination and a plane that is at least in contact with the jaw bone.
[27] The program according to [22],
   wherein the movement information generation unit generates the information indicating the intrusion amount or the extrusion amount of jaw bones as a length between a position of a jaw bone before movement and a position of the jaw bone when assumed to be moved, in a direction perpendicular to a reference plane serving as a reference for movement.
[28] The program according to [22],
   wherein the movement information generation unit generates the information indicating the translational movement amount of jaw bones as a length between a position of a jaw bone before movement and a position of the jaw bone when assumed to be moved, in a direction along a reference plane serving as a reference for movement.
[29] The program according to any one of [21] to [28],
   wherein the computer is further caused to function as a position identification support unit configured to generate position information generation support information for supporting identification of positions of anterior teeth, molars, or jaw bones of the subject by using the measurement point information acquired by the measurement point information acquisition unit,
   and wherein the position information acquisition unit acquires the imaging-time position information based on user input using the position information generation support information generated by the position identification support unit.
[30] The program according to any one of [21] to [28],
   wherein the measurement point information acquisition unit acquires the measurement point information based on a standardized head X-ray photograph, which is an image obtained by imaging a subject's head X-ray.

### Effects of the Invention

According to the present invention, it is possible to provide a novel technology that can be used to support dental treatment.

### Brief Description of the Drawings

FIG. 1 is a diagram illustrating an example configuration of a first embodiment.
FIG. 2 is a diagram illustrating an example configuration of a personal computer 1 according to the first embodiment.
FIG. 3 is a diagram illustrating functional blocks of the first embodiment.
FIG. 4 is a diagram illustrating a processing flow related to generation of measurement point information and trace line information in the first embodiment.
FIG. 5 is a diagram illustrating an example screen related to generation of measurement point information and trace line information in the first embodiment.
FIG. 6 is a diagram illustrating an example screen related to generation of measurement point information and trace line information in the first embodiment.
FIG. 7 is a diagram illustrating an example screen related to generation of measurement point information and trace line information in the first embodiment.
FIG. 8 is a diagram illustrating an example screen related to generation of measurement point information and trace line information in the first embodiment.
FIG. 9 is a diagram illustrating a processing flow related to generation of movement information in the first embodiment.
FIG. 10 is a diagram illustrating an example screen related to generation of tooth movement information in the first embodiment.
FIG. 11 is a diagram illustrating an example screen related to generation of tooth movement information in the first embodiment.
FIG. 12 is a diagram illustrating an example screen related to generation of tooth movement information in the first embodiment.
FIG. 13 is a diagram illustrating an example screen related to generation of tooth movement information in the first embodiment.
FIG. 14 is a diagram illustrating an example screen related to generation of tooth movement information in a second embodiment.
FIG. 15 is a diagram illustrating a processing flow related to generation of movement information in the second embodiment.
FIG. 16 is a diagram illustrating a processing flow related to generation of movement information in the second embodiment.
FIG. 17 is a diagram illustrating a processing flow related to generation of movement information in the second embodiment.
FIG. 18 is a diagram illustrating a three-dimensional image obtained by imaging a subject's head X-ray, which can be used in other embodiments.

### Description of Embodiments

### [First Embodiment]

Hereinafter, one embodiment of the present invention will be described in detail. The first embodiment relates to a dental treatment support system, and includes a measurement point information acquisition unit, a position information acquisition unit, a planned position information acquisition unit, and a movement information generation unit.

The measurement point information acquisition unit acquires measurement point information indicating measurement points corresponding to parts of a head, based on a standardized head X-ray photograph of a subject.

The position information acquisition unit acquires, based on the measurement point information, imaging-time position information indicating positions of anterior teeth or molars of the subject at the time of imaging.

The planned position information acquisition unit acquires planned position information indicating positions when the anterior teeth or molars are assumed to be moved.

The movement information generation unit generates movement information indicating an amounts of movement from positions of the anterior teeth or molars before movement to positions when the anterior teeth or molars are assumed to be moved, based on the measurement point information, the imaging-time position information, and the planned position information.

In addition, the dental treatment support system of the first embodiment further includes a position identification support unit configured to generate position information generation support information for supporting identification of positions of teeth of the subject by using the measurement point information acquired by the measurement point information acquisition unit. The position information acquisition unit acquires the imaging-time position information based on user input using the position information generation support information generated by the position identification support unit.

Herein, in the present specification, a "standardized head X-ray photograph" means a two-dimensional standardized X-ray photograph obtained by imaging a head from a lateral side with a midsagittal plane as a reference plane, and is also referred to as a cephalogram.

In addition, in the present specification, a "measurement point," also referred to as a landmark, means a reference point corresponding to each region included in the head. A measurement point is also used to represent a plane in which two or more measurement points exist, or a plane in which reference points derived from such two or more measurement points exist. The measurement point information includes information regarding measurement points for which positions are set for each subject.

As measurement points, for example, those used in cephalometric analysis can also be used in the dental treatment support system of the first embodiment. Specific measurement points include S (Sella turcica, center of the sella turcica), N (Nasion, most anterior point of the frontonasal suture), Or (Orbitale, lowest point of the orbital rim), ANS (Anterior Nasal Spine, tip of the anterior nasal spine), U1 (Upper 1, incisal edge of the maxillary central incisor), L1 (Lower 1, incisal edge of the mandibular central incisor), Po (Porion, upper margin point of the bony external auditory meatus), Pog (Pogonion, most protruding point of the mandibular symphysis), PNS (Posterior Nasal Spine, most posterior point of the posterior nasal spine), Me (Menton, lowest point on the midsagittal section of the mandibular symphysis), D (D point, central point of the mandibular symphysis with respect to the SN plane), UMo (Upper Molar, most distal point of the crown of the maxillary first molar), LMo (Lower Molar, central point of the occlusal surface of the mandibular first molar), Go (Gonion, a point at which an angle bisector of an angle formed by the mandibular plane and the posterior border plane of the ramus intersects the mandibular angle), and the like.

In addition, as a plane in which two or more measurement points exist, or a plane in which reference points derived from such two or more measurement points exist, mention may be made, for example, of an SN plane (a plane including S and N), a Frankfurt plane (FH plane, a plane including Or and Po), an occlusal plane (a plane including a midpoint between UMo and LMo and a midpoint between U1 and L1), a palatal plane (a plane including ANS and PNS), a mandibular plane (a plane including Go and Me), and the like.

These are described, for example, in Hiroyuki Muramatsu, "Techniques for Reading Cephalograms That Improve Diagnostic Ability," Quintessence Publishing, published Jun. 10, 2010, and Akira Kameda, "New Edition: Dictionary of Orthodontics," Quintessence Publishing, published Jan. 10, 2018.

Note that a standardized head X-ray photograph is a two-dimensional image, and the above planes are also represented as lines; however, in the field of orthodontics, for example, these are described as "planes" even when represented as lines. Therefore, in the present specification as well, a "plane" is a concept including a line represented in a two-dimensional image, and is collectively referred to as a plane.

The imaging-time position information includes information indicating positions of teeth at the time of imaging of the standardized head X-ray photograph. Specifically, as the imaging-time position information, mention may be made of trace line information indicating trace lines generated based on the standardized head X-ray photograph, which will be described later. In addition, the planned position information includes information indicating positions of teeth when the teeth are assumed to be moved by treatment. Specifically, as the planned position information, mention may be made of information indicating an instruction regarding a position to which the teeth are to be moved, which will be described later.

The movement information is information indicating amounts of movement from positions of teeth before movement when the teeth are assumed to be moved, and a unit of the movement amount is a unit relating to length (e.g., mm) or a unit of angle (e.g., degrees).

Examples of teeth for which the movement information is generated include anterior teeth (upper and lower anterior teeth) and/or molars (upper and lower molars). The upper and lower anterior teeth refer to six maxillary teeth and six mandibular teeth from the front to the left and right canines. The movement information of anterior teeth can be generated for one or two or more among these six maxillary teeth and six mandibular teeth.

In addition, the upper and lower molars refer to four maxillary teeth and four mandibular teeth: a first premolar, a second premolar, a first molar, and a second molar. The movement information of molars can be generated for one or two or more among these four maxillary teeth and four mandibular teeth.

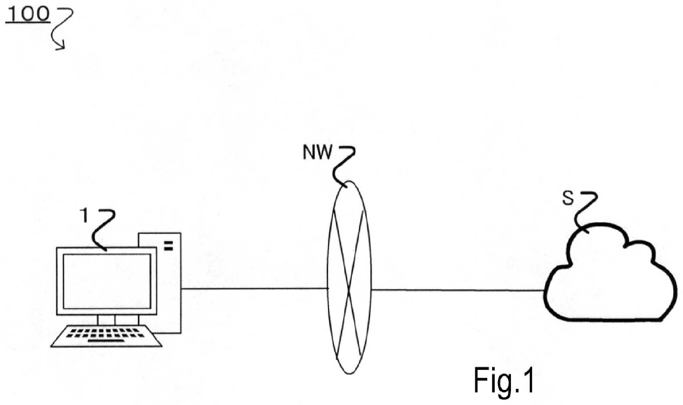

FIG. 1 is a schematic diagram of a dental treatment support system 100 of the first embodiment. The dental treatment support system 100 of the first embodiment includes a personal computer 1 and a server S connected to the personal computer 1 so as to be communicable with each other via a network NW. The server S may be, for example, a cloud-based type, and embodiments such as an on-premises type may also be adopted as appropriate.

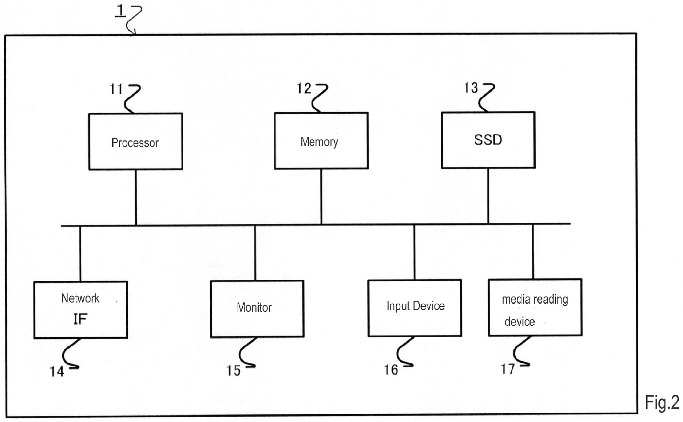

FIG. 2 is a schematic diagram of the personal computer 1 according to the first embodiment. The personal computer 1 includes a processor 11 as an arithmetic processing device, a memory 12 as a main storage device, and an SSD (Solid State Drive) 13 as an auxiliary storage device. Note that an HDD (Hard Disk Drive) may be used instead of the SSD, and there is no particular limitation. The dental treatment support system 100 also includes a network IF (interface) 14 that controls communication of external units, a monitor 15, input devices 16 (e.g., a keyboard and a mouse), and a media reading device 17.

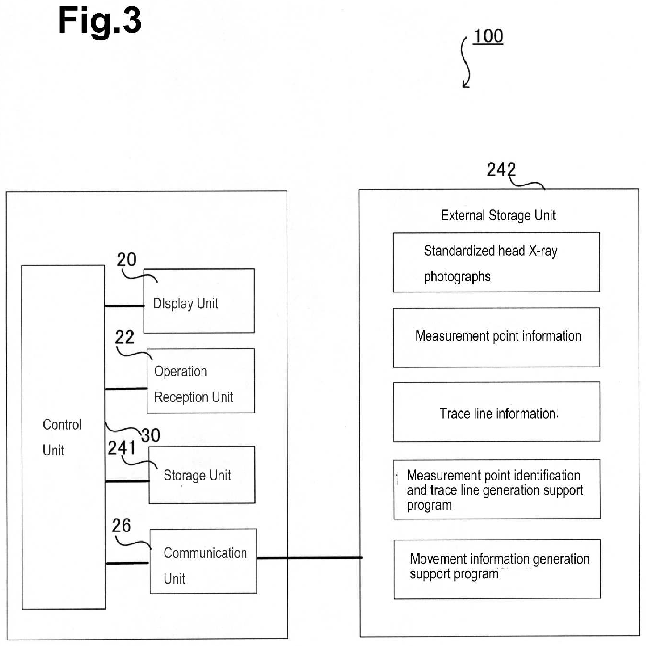

FIG. 3 is a block diagram of the dental treatment support system 100 of the first embodiment. As shown in FIG. 2, the dental treatment support system 100 includes a display unit 20 that displays various types of information, an operation reception unit 22 that receives operations of a user such as a doctor, a storage unit 241 and an external storage unit 242 that store various types of information, a communication unit 26 connected to the network NW, and a control unit 30 that controls operations of these units.

Among these, the display unit 20, the operation reception unit 22, the storage unit 241, the communication unit 26, and the control unit 30 can be implemented by the personal computer 1, and the external storage unit 242 can be implemented by the server S.

Specifically, the display unit 20 is composed of, for example, the monitor 15 shown in FIG. 2, and has a function of displaying various types of information in response to a control signal from the control unit 30.

In addition, as shown in FIG. 2, the operation reception unit 22 is composed of, for example, input devices 16 such as a keyboard, a mouse, or a touch panel that also serves as the display unit 20, and has a function of receiving an input operation by the user and a function of outputting, to the control unit 30, information indicating the received input content.

In addition, the storage unit 241 is composed of, for example, the memory 12, the SSD 13, and the like, and has a function of writing and storing various types of information and a function of reading various types of information.

The external storage unit 242 is composed of the server S, and has a function of receiving requests from computers/terminals connected via the network NW, writing various types of information in response to the requests, and sending back information responsive to the requests to the respective computers/terminals.

In the first embodiment, the external storage unit 242 stores image data of standardized head X-ray photographs for each subject, a measurement point identification and trace line generation support program, a movement information generation support program, generated measurement point information, trace line information, and the like.

In addition, the communication unit 26 is composed of the network IF 14.

In addition, the control unit 30 is composed of hardware such as the processor 11 and the memory 12, and software such as a control program.

The control unit 30 has a processing function related to transmission and reception of various signals with the display unit 20, the operation reception unit 22, the storage unit 241, the external storage unit 242, and the communication unit 26, and a function of controlling operations of respective units connected via a predetermined bus.

In the first embodiment, the control unit 30, the display unit 20, and the operation reception unit 22 correspond to the measurement point information acquisition unit, the position information acquisition unit, and the planned position information acquisition unit. In addition, the control unit 30 corresponds to the movement information generation unit and the position identification support unit.

Next, a processing flow in the first embodiment will be described.

In the following description, an embodiment in which movement information is generated for one or two or more among a maxillary central incisor, a mandibular central incisor, a maxillary first molar, and a mandibular first molar will be described as an example.

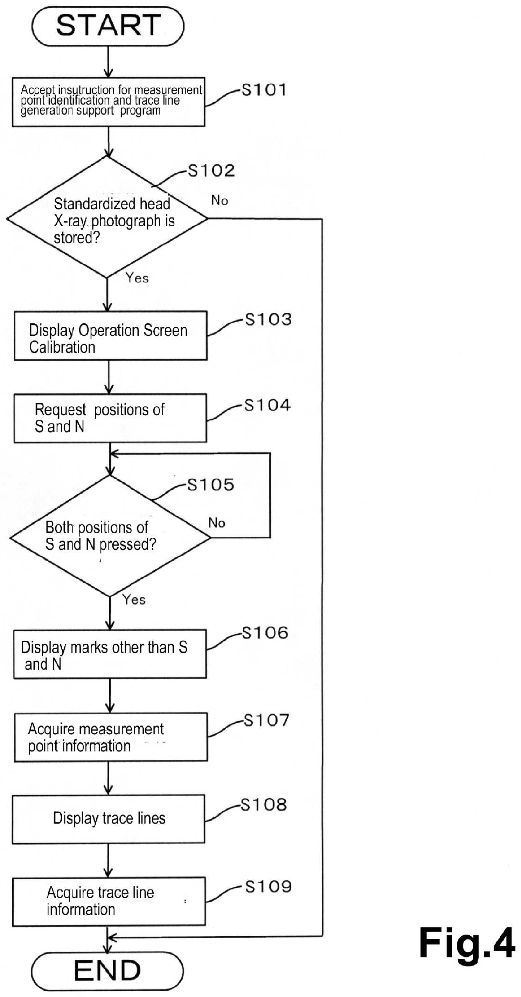

First, a processing flow related to measurement point setting and trace line generation shown in FIG. 4 will be described.

First, in step S101, when an operation (start instruction) by a user for executing a measurement point identification and trace line generation support program is accepted, the control unit 30 of the apparatus 100 executes the program and enables acceptance of user operations.

In step S102, the control unit 30 determines whether a standardized head X-ray photograph is stored in the external storage unit 242.

If the standardized head X-ray photograph is not stored in the external storage unit 242, the control unit 30 terminates the processing.

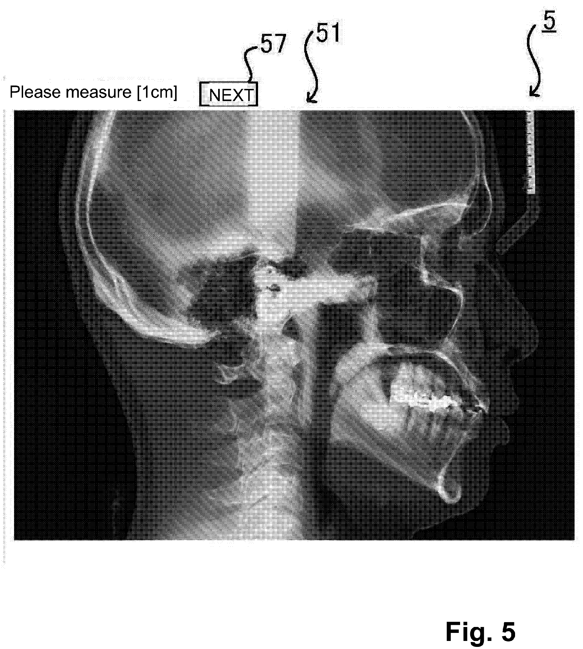

On the other hand, if the standardized head X-ray photograph is stored in the external storage unit 242, the control unit 30 displays, on the display unit 20, an operation screen 5 for accepting a user operation related to identification of measurement points as shown in FIG. 5 (step S103).

On the operation screen 5, as shown in FIG. 5, the standardized head X-ray photograph is displayed in an image display area 51 on the left side of the screen.

In step S103, the control unit 30 displays, on the operation screen 5, a message "Please measure [1 cm]," and accepts an instruction from the user for size adjustment (calibration; matching the ratio of the screen with the ratio of the photograph). Input is performed by pressing a button 57 labeled "Next." Based on the input of the instruction, the control unit 30 performs processing related to size adjustment.

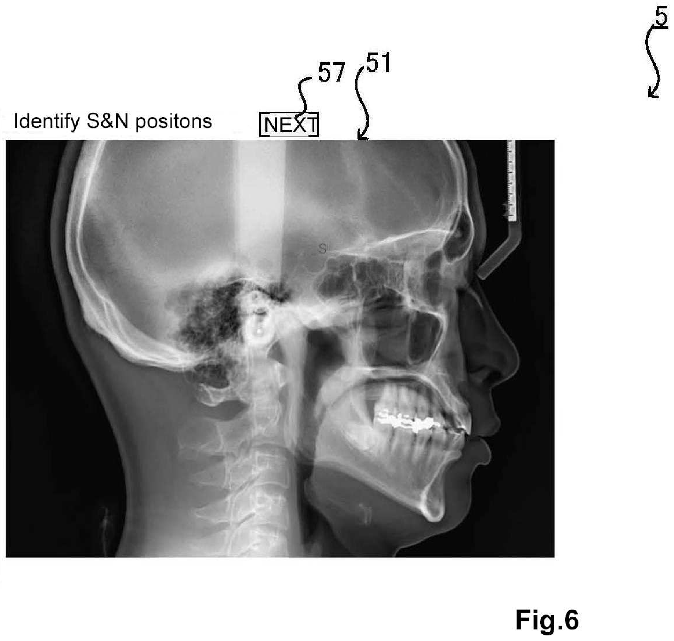

In step S104, as shown in FIG. 6, the control unit 30 displays, on the operation screen 5, a message "Please specify the positions of S and N."

In step S105, the control unit 30 determines whether any two positions within the image display area 51 have been pressed.

If two positions have not been pressed, the control unit 30 does not perform processing and waits.

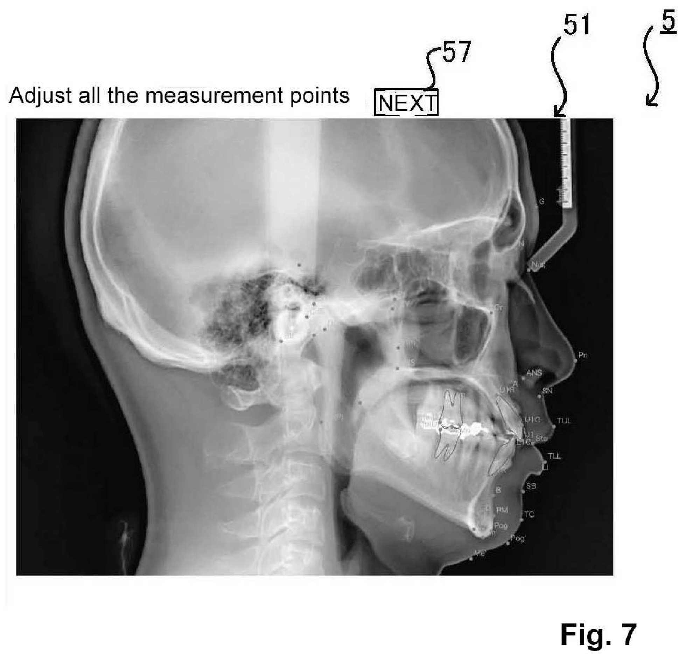

On the other hand, if any two positions within the image display area 51 have been pressed, the control unit 30, as shown in FIG. 7, displays circular marks corresponding to S and N superimposed on the pressed positions on the standardized head X-ray photograph, and also displays, as provisional positions, circular marks indicating measurement points other than S and N superimposed on positions on the standardized head X-ray photograph that are assumed to correspond thereto (step S106).

The user visually determines whether the positions of the circular marks are displayed at desired positions, and if any circular mark differs from a desired position, performs an operation to move the circular mark. The control unit 30 acquires, via the operation reception unit 22, information indicating an instruction by the operation, and corrects positions at which the circular marks are displayed.

When all of the circular marks are displayed at desired positions, the user presses a button for inputting confirmation of measurement point positions (button 57 labeled "Next" in the screen 5 shown in FIG. 7).

When position confirmation is input by pressing the button, the control unit 30 acquires coordinate information indicating the positions of the circular marks on the standardized head X-ray photograph as measurement point information indicating the positions of measurement points (step S107).

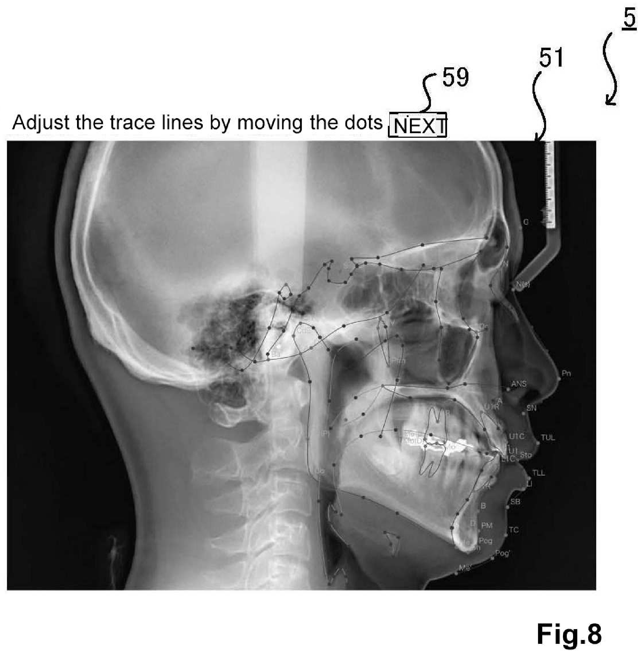

In step S108, as shown in FIG. 8, based on the measurement point information, the control unit 30 displays, superimposed on the standardized head X-ray photograph as provisional positions, lines (trace lines) indicating positions of teeth and a facial contour. Note that the trace lines displayed at the provisional positions correspond to position information generation support information.

The user visually determines whether the trace lines are displayed at desired positions, and if any trace line differs from a desired position, performs an operation to move or deform the line. Specifically, the trace line is moved or deformed by operating a point on the trace line. The control unit 30 acquires, via the operation reception unit 22, information indicating an instruction by the operation, and corrects positions at which the trace lines are displayed.

If the trace lines are displayed at desired positions, the user presses a button for inputting confirmation of trace line positions (button 59 in the screen 5 shown in FIG. 8, on which a message "Complete" is displayed).

When confirmation of the trace line positions is input by pressing the button, the control unit 30 acquires the trace lines on the standardized head X-ray photograph as imaging-time trace line information (corresponding to imaging-time position information), stores the imaging-time trace line information in the external storage unit 242, and terminates the processing (step S109).

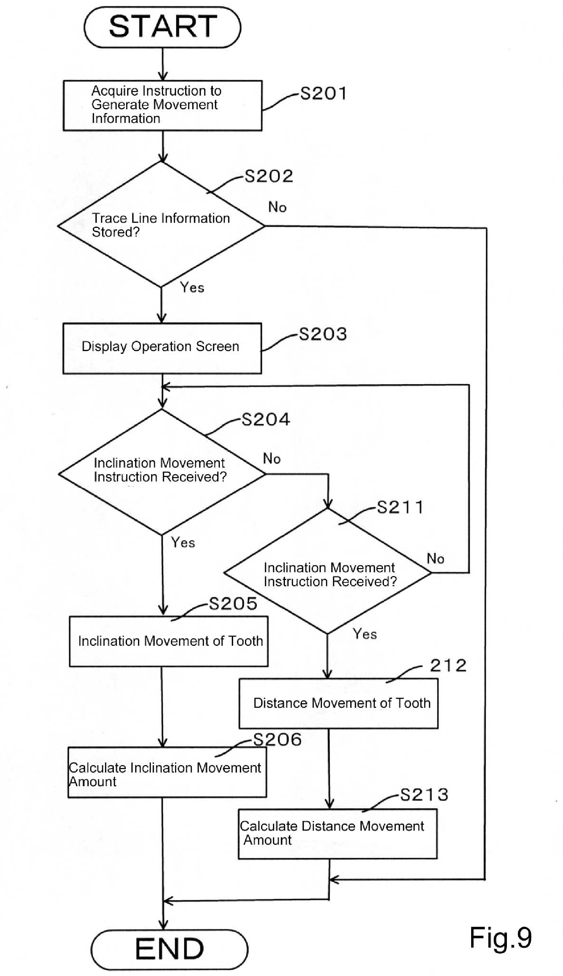

Next, a processing flow related to movement information generation shown in FIG. 9 will be described.

First, in step S201, when an operation (start instruction) by a user for executing a movement information generation support program is accepted, the control unit 30 of the apparatus 100 executes the program and enables acceptance of user operations.

In step S202, the control unit 30 determines whether the standardized head X-ray photograph, the measurement point information, and the trace line information are stored in the external storage unit 242.

If the standardized head X-ray photograph is not stored in the external storage unit 242, or if only the standardized head X-ray photograph is stored in the external storage unit 242, the control unit 30 terminates the processing.

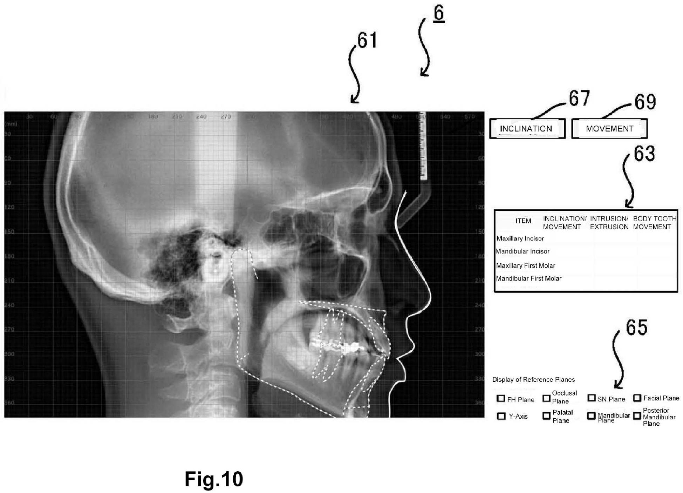

On the other hand, if the standardized head X-ray photograph, the measurement point information, and the trace line information are stored in the external storage unit 242, the control unit 30 displays, on the display unit 20, an operation screen 6 for accepting a user operation related to movement information generation as shown in FIG. 10 (step S203).

On the operation screen 6, as shown in FIG. 10, the standardized head X-ray photograph is displayed in an image display area 61 on the left side of the screen, and the trace lines are displayed superimposed on the standardized head X-ray photograph.

In addition, 63 is a table indicating movement amounts.

In addition, 65 is a plurality of checkboxes for inputting an instruction to display, superimposed on the standardized head X-ray photograph, lines indicating planes based on the measurement point information.

In addition, 67 is a button for inputting an instruction to generate an inclination movement amount of a tooth, and 69 is a button for inputting an instruction to generate an intrusion amount or an extrusion amount of a tooth, and an instruction to generate a translational movement amount of a tooth. Hereinafter, intrusion or extrusion, and translational movement are collectively also referred to as distance movement.

In step S204, the control unit 30 determines whether an instruction to generate an inclination movement amount of a tooth as movement information has been input by pressing the button 67 labeled "Tooth Inclination" on the operation screen 6.

If it is determined that an instruction to generate an inclination movement amount of a tooth has not been input, the control unit 30 determines whether an instruction to generate a distance movement amount of a tooth as movement information has been input by pressing a button 69 labeled "Tooth Movement" on the operation screen 6 (step S211).

If it is determined in step S211 that an instruction to generate a distance movement amount of a tooth has not been input, the control unit 30 does not perform processing and returns to step S204.

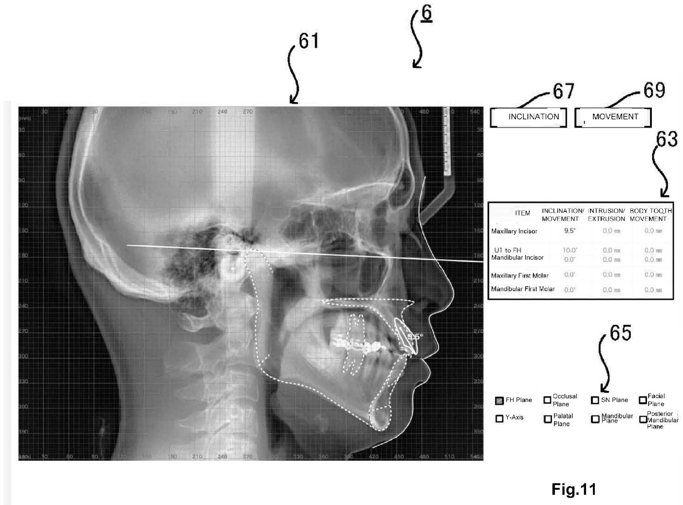

If it is determined in step S204 that an instruction to generate an inclination movement amount of a tooth has been input, the control unit 30, as shown in FIG. 11, acquires, via the operation reception unit 22, information indicating an instruction regarding a position to which the tooth is to be moved as planned position information, and deforms the trace line to display movement of the tooth (step S205).

In FIG. 11, a position of the tooth before movement is indicated by a broken line, and a position of the tooth when assumed to be moved is indicated by a solid line.

In addition, based on the trace line information, the planned position information, and the measurement point information stored in the external storage unit 242, the control unit 30 calculates a tooth movement amount (inclination movement amount) using an angle as a unit, and displays the calculated amount in the table 63 on the operation screen 6 (step S206).

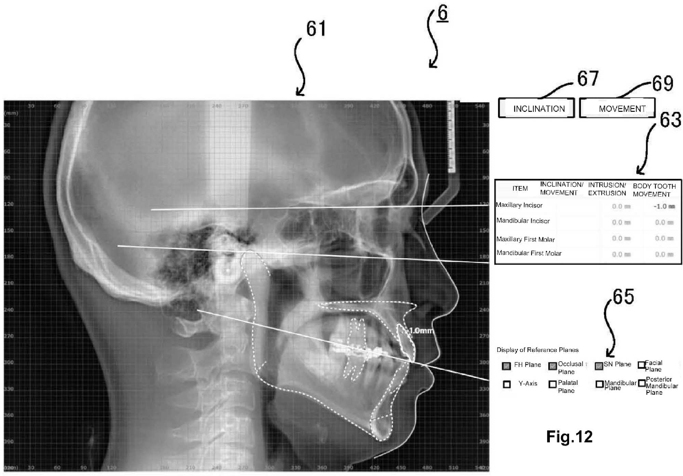

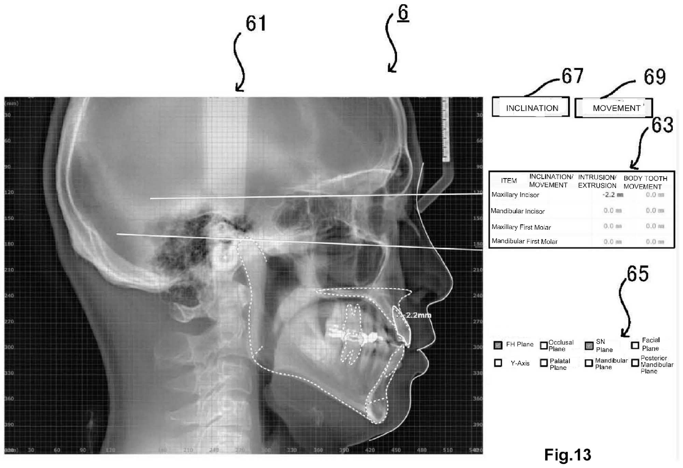

If it is determined in step S211 that an instruction to generate a distance movement amount of a tooth has been input, the control unit 30, as shown in FIGS. 12 and 13, acquires, via the operation reception unit 22, information indicating an instruction regarding a position to which the tooth is to be moved as planned position information, and deforms the trace line to display movement of the tooth (step S212).

In addition, based on the trace line information, the planned position information, and the measurement point information stored in the external storage unit 242, the control unit 30 generates, using a length as a unit, an intrusion amount or an extrusion amount of the tooth and/or a translational movement amount of the tooth, and displays the generated amounts in the table 63 on the operation screen 6 (step S213).

Note that, for calculation of movement amounts, anatomical structures in the maxillofacial region that are depicted in standardized head X-ray photographs and have high reproducibility, such as a Frankfurt plane, an SN plane, an occlusal plane, a mandibular plane, and a palatal plane, which are commonly used in cephalometric analysis, are used as references.

Specific processing for such calculation can also be performed by execution of the movement information generation support program, and measurement points and reference planes used for the calculation can also be preset in the program.

For example, an inclination movement amount of a tooth can be generated as a change amount in an angle of a tooth axis relative to a reference plane serving as a reference for inclination.

Specifically, an inclination movement amount of a tooth (unit: angle) can be obtained by calculating a change amount in an angle, relative to the reference plane, of a tooth axis before movement (at the time of imaging) and a tooth axis when the tooth is assumed to be moved. The tooth axis is an axis extending along a direction in which the tooth grows, i.e., a long axis of the tooth. For example, for a maxillary central incisor, the tooth axis is a straight line on which a measurement point U1 and a measurement point U1R (Upper 1 Root, root apex of the maxillary central incisor) lie; for a mandibular central incisor, the tooth axis is a straight line on which a measurement point L1 and a measurement point L1R (Lower 1 Root, root apex of the mandibular central incisor) lie.

The reference plane can be appropriately set in consideration of the tooth type and the like, and examples thereof include a Frankfurt plane, an SN plane, an occlusal plane, a mandibular plane, and a palatal plane. Which reference plane is to be used can be preset in, for example, the movement information generation support program.

In addition, an inclination movement amount can also be calculated solely based on a change in an angle of the tooth axis before movement and when assumed to be moved. In FIG. 11, such a value (for example, maxillary anterior tooth: 9.5° in FIG. 11) is also displayed. On the other hand, by performing calculation using the above-described reference plane, it is also possible to evaluate inclination movement of anterior teeth or molars relative to the skull obtained by a treatment plan, which is preferable.

For example, in FIG. 11, an angle of a tooth axis of a maxillary central incisor before movement relative to an FH plane selected as a reference plane is 110.68 degrees, and an angle of the tooth axis of the maxillary central incisor when assumed to be moved relative to the FH plane is 120.68 degrees.

In this case, it is calculated that the maxillary central incisor has been inclined by 10 degrees toward a labial side (displayed as "U1 to FH: 10°" in FIG. 11).

In addition, a translational movement amount of a tooth can be generated as a length between a position of the tooth before movement and a position of the tooth when assumed to be moved, in a direction along a reference plane serving as a reference for movement.

Specifically, a translational movement amount of a tooth (unit: length) can be calculated, using a preset reference plane (X-axis) and a predetermined portion (reference point) of the tooth, as an amount by which the reference point of the tooth moves in a direction parallel to the reference plane between before movement and when the tooth is assumed to be moved.

The reference plane may be, for example, a Frankfurt plane, an SN plane, an occlusal plane, a mandibular plane, or a palatal plane.

In addition, the reference point of the tooth can be appropriately set depending on the tooth type; for example, a center of the tooth root can be used. The center of the tooth root is also referred to as a center of rotation (center of resistance), and is generally defined as 1/2 to 1/3 from an apex side of the tooth root. For example, the center of the tooth root may be preset as "center of tooth root: 1/2 from the root apex" in the movement information generation support program, and a specific position thereof can be defined using the trace line information indicating the position of the tooth at the time of imaging.

Note that, in calculating a translational movement amount, a position before movement may be set as 0, movement toward an anterior (labial) side may be assigned a positive sign (+), and movement toward a posterior (lingual) side may be assigned a negative sign (-), thereby distinguishing directions.

For example, in FIG. 12, a case will be described in which a reference plane preset in the movement information generation support program is an occlusal plane and a translational movement amount is generated based on a center of a tooth root of a maxillary central incisor. In this case, in FIG. 12, it is calculated that the tooth has moved 10 mm toward a lingual side along the occlusal plane after the tooth has been moved.

In addition, an intrusion amount or an extrusion amount of a tooth is generated as a length between a position of the tooth before movement and a position of the tooth when assumed to be moved, in a direction perpendicular to a reference plane serving as a reference for movement.

Specifically, an intrusion amount or an extrusion amount of a tooth (unit: length) can be obtained by calculating a length in a direction perpendicular to the reference plane between a predetermined portion (reference point) of the tooth before movement and the reference point of the tooth when assumed to be moved.

At this time, a case where the tooth moves toward a root side may be expressed as an intrusion amount (for example, with a negative sign (-) assigned with a pre-movement position set as 0), and a case where the tooth moves toward a crown side may be expressed as an extrusion amount (for example, with a positive sign (+) assigned with a pre-movement position set as 0).

The reference plane can be set according to the tooth type and the like, and may be, for example, an FH plane (Frankfurt plane), a mandibular plane, or a palatal plane.

In addition, the reference point of the tooth is not particularly limited and can be set according to the tooth type and the like, and examples thereof include a tooth crown tip (represented, based on measurement points, as U1 for a maxillary central incisor, L1 for a mandibular central incisor, UMo for a maxillary first molar, and LMo for a mandibular first molar), and a center of a tooth root (represented as CR based on measurement points).

For example, in FIG. 13, a case will be described in which an intrusion amount or an extrusion amount is calculated based on a center of a tooth root of a maxillary central incisor with an FH plane as a reference plane. In the example of FIG. 13, since the tooth moves toward a root side in a direction perpendicular to the FH plane, an intrusion movement amount of -2.2 mm is calculated.

In addition, an incisal edge, a root, a crown, a cervical area, a gingival margin, contact points of teeth, an occlusal surface of a tooth, an interdental papilla, a gingival sulcus, an alveolar crest, an apex of the root, an apical foramen, a center of rotation for inclination of a tooth, and the like may also be used as reference points for a translational movement amount of a tooth and for an intrusion amount or an extrusion amount of a tooth. Positions of these reference points can also be defined based on the measurement point information or the trace line information.

In addition, which reference point and which reference plane are to be used for the translational movement amount of a tooth and for the intrusion amount or the extrusion amount of a tooth can be preset in, for example, the movement information generation support program.

As described above, according to the first embodiment, for example, when planning orthodontic treatment, a treatment policy can be formulated more rapidly and more accurately. In addition, monitoring of treatment progress can also be performed more efficiently. Furthermore, for example, contributions can be expected to improvement of an overall workflow by simplifying setup instructions to dental technicians and to enhancement of collaboration among doctors.

### [Second Embodiment]

Next, a second embodiment will be described. Components common to those of the first embodiment are denoted by the same reference numerals, and description thereof will be omitted.

In the second embodiment, movement information can be generated not only for anterior teeth and molars but also for jaw bones (maxillary and mandibular bones). This makes it easier to formulate a treatment plan even when, for example, surgical orthodontic treatment is performed.

In the second embodiment, the imaging-time position information includes information indicating positions of jaw bones in addition to information indicating positions of teeth at the time of imaging of the standardized head X-ray photograph. Note that, although information indicating positions of jaw bones is also acquired as imaging-time position information in the first embodiment already described, in the first embodiment, information indicating positions of jaw bones does not necessarily have to be acquired.

In addition, in the second embodiment, the position information generation support information includes information indicating provisional positions of jaw bones in addition to information indicating provisional positions of teeth. Note that, although the position information generation support information includes information indicating provisional positions of jaw bones in the first embodiment already described, in the first embodiment, information indicating provisional positions of jaw bones does not necessarily have to be included. In addition, in the second embodiment, the planned position information may include information indicating positions of jaw bones when the jaw bones are assumed to be moved by treatment, in addition to information indicating positions of teeth when the teeth are assumed to be moved by treatment. Specifically, the planned position information may include information indicating an instruction regarding a position to which teeth are to be moved and information indicating an instruction regarding a position to which jaw bones are to be moved.

In addition, in the second embodiment, the movement information may include, in addition to information indicating amounts of movement from positions of teeth before movement when the teeth are assumed to be moved, information indicating amounts of movement from positions of jaw bones before movement when the jaw bones are assumed to be moved.

In addition, also in the second embodiment, which reference point and which reference plane are to be used can be preset in, for example, the movement information generation support program.

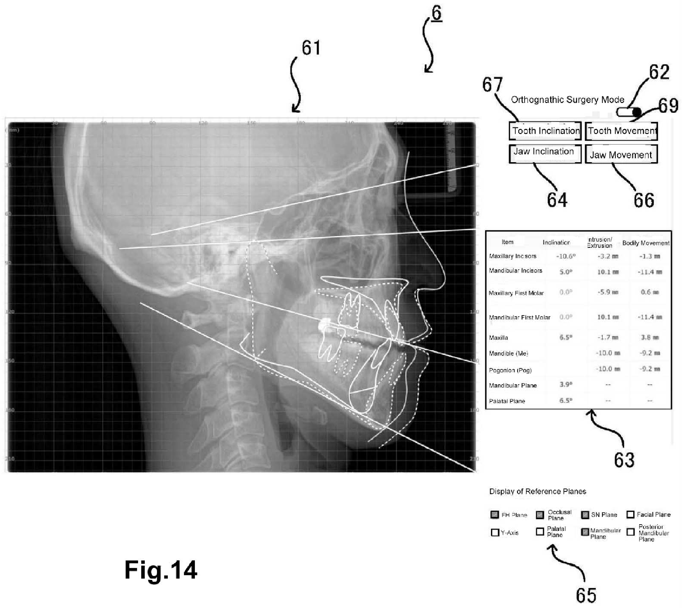

FIG. 14 is a diagram illustrating an operation screen related to movement information generation in the second embodiment.

The operation screen 6 includes, similarly to the first embodiment, an image display area 61 in which a standardized head X-ray photograph is displayed, a table 63 indicating movement amounts, a button 67 for inputting an instruction to generate an inclination movement amount of a tooth, and a button 69 for inputting an instruction to generate a movement amount of a tooth.

In addition, in the second embodiment, the operation screen 6 further includes a toggle button 62 for inputting an instruction to execute processing for generating movement amounts also for jaw bones (hereinafter also referred to as a surgical orthodontic mode), a button 64 for inputting an instruction to generate an inclination movement amount of a jaw bone, and a button 66 for inputting an instruction to generate a movement amount of a jaw bone.

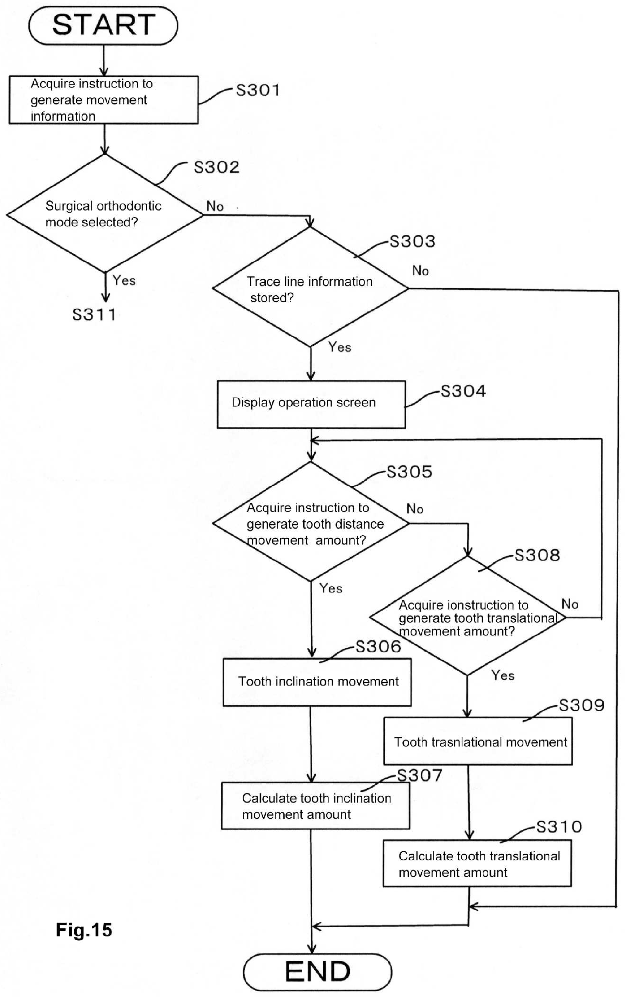

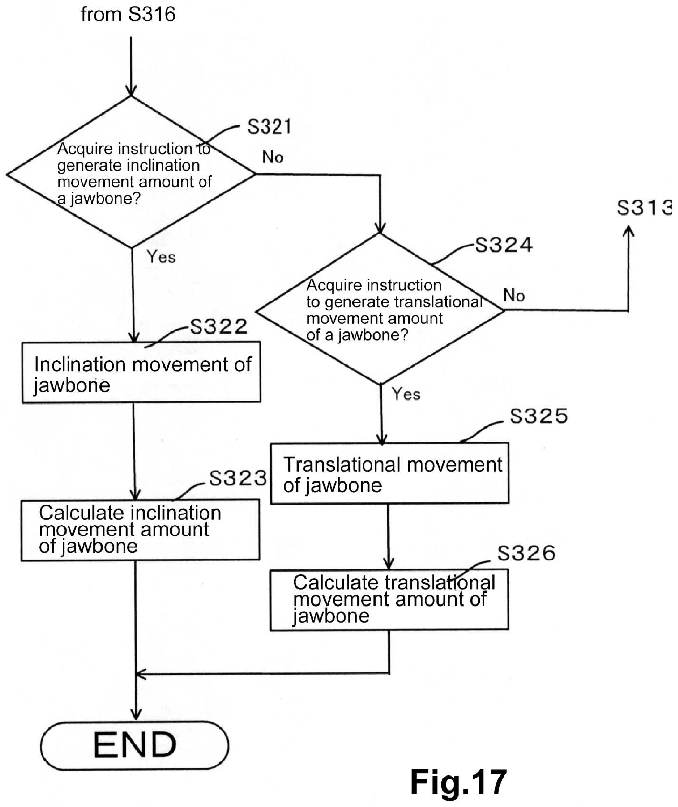

A processing flow related to movement information generation in the second embodiment shown in FIGS. 15 to 17 will be described.

First, in step S301, when an operation (start instruction) by a user for executing the movement information generation support program is accepted, the control unit 30 of the apparatus 100 executes the program and enables acceptance of user operations.

In step S302, the control unit 30 determines whether an instruction to execute the surgical orthodontic mode has been input by an operation of the toggle button 62 on the operation screen 6.

If it is determined that the instruction to execute the surgical orthodontic mode has not been input, the process proceeds to step S303, and processing is performed. Note that the processing of steps S303 to S310 is common to the processing of steps S202 to S206 and steps S211 to S213 related to movement information generation in the first embodiment, and therefore description thereof will be omitted.

If it is determined in step S302 that the instruction to execute the surgical orthodontic mode has been input, the process proceeds to step S311.

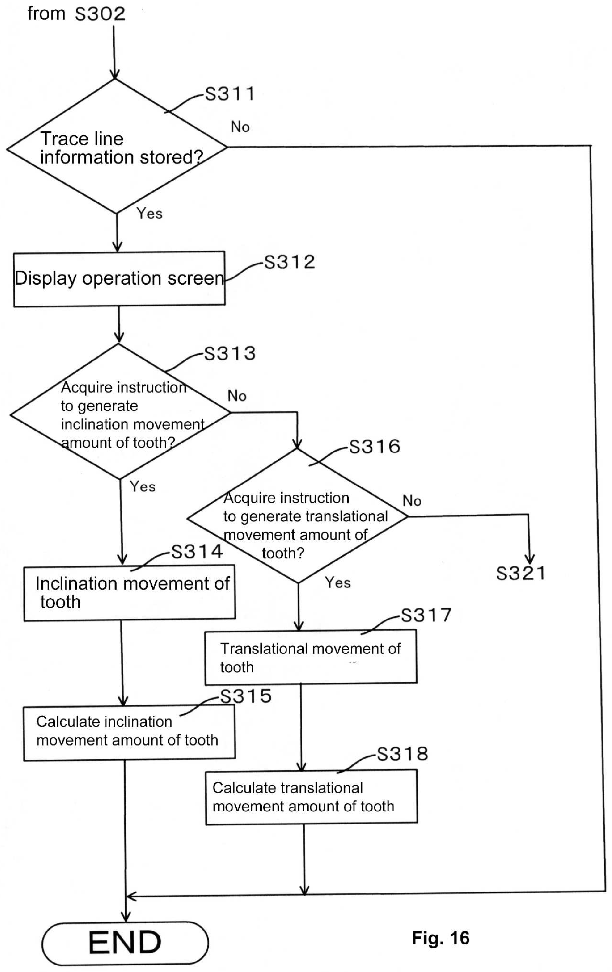

Processing of steps S311 to S315 and steps S317 and S318 in FIG. 16 is common to the processing of steps S202 to S206 and steps S212 and S213, and therefore description thereof will be omitted.

If it is determined in step S316 that an instruction to generate a distance movement amount of a tooth has not been input, the control unit 30 proceeds to step S321 and determines whether an instruction to generate an inclination movement amount of a jaw bone has been input. If it is determined in step S316 that an instruction to generate a distance movement amount of a tooth has been input, the control unit 30 proceeds to step S317 and performs processing.

If it is determined in step S321 that an instruction to generate an inclination movement amount of a jaw bone has been input, the control unit 30 acquires, via the operation reception unit 22, information indicating an instruction regarding a position to which the jaw bone is to be moved, deforms the trace line to display movement of the jaw bone (step S322), and calculates an inclination movement amount of the jaw bone using an angle as a unit based on the trace line information, the information indicating an instruction regarding the position to which the jaw bone is to be moved, and the measurement point information stored in the external storage unit 242, and displays the calculated amount in the table 63 on the operation screen 6 (step S323).

On the other hand, if it is determined in step S321 that an instruction to generate an inclination movement amount of a jaw bone has not been input, the process proceeds to step S324, and the control unit 30 determines whether an instruction to generate a distance movement amount of a jaw bone has been input. If it is determined that the instruction has not been input, the control unit 30 returns to step S313 and waits for an instruction to be input.

If it is determined in step S324 that an instruction to generate a distance movement amount of a jaw bone has been input, the control unit 30 acquires, via the operation reception unit 22, information indicating an instruction regarding a position to which the jaw bone is to be moved, deforms the trace line to display movement of the jaw bone (step S325), and calculates a distance movement amount of the jaw bone using a length as a unit based on the trace line information, the information indicating an instruction regarding the position to which the jaw bone is to be moved, and the measurement point information stored in the external storage unit 242, and displays the calculated amount in the table 63 on the operation screen 6 (step S326).

Also for calculation of movement amounts of jaw bones, anatomical structures in the maxillofacial region that are depicted in standardized head X-ray photographs and have high reproducibility, such as a Frankfurt plane, an SN plane, an occlusal plane, a mandibular plane, and a palatal plane, which are commonly used in cephalometric analysis, can be used as references.

An inclination movement amount (unit: angle) is generated as a change amount in an angle between a reference plane serving as a reference for inclination and a plane that is at least in contact with the jaw bone.

Specifically, an inclination movement amount of a jaw bone (unit: angle) can be obtained by calculating a change amount in an angle, relative to the reference plane, of a plane that is at least in contact with the jaw bone before movement (at the time of imaging) and when the jaw bone is assumed to be moved.

In the present specification, a "plane that is at least in contact with the jaw bone" means a plane that intersects the jaw bone or is in contact with the jaw bone at one point or at two or more points.

Examples of the reference plane include a Frankfurt plane and an SN plane.

Examples of the plane that is at least in contact with the jaw bone include, for example, a palatal plane (in the case of a maxillary bone) and a mandibular plane (in the case of a mandibular bone).

The present invention is not limited thereto, and an inclination movement amount may be calculated based solely on a change in an angle of a plane that is at least in contact with the jaw bone before movement and after movement.

Although an inclination movement amount can be calculated solely based on the change in angle of the plane that is at least in contact with the jaw bone before movement and after movement, calculation using the above-described reference plane is preferable because it also allows evaluation of an angle of the maxillary bone or the mandibular bone relative to the skull obtained by a treatment plan.

A translational movement amount of a jaw bone (unit: length) is generated as a length between a position of the jaw bone before movement and a position of the jaw bone when assumed to be moved, in a direction along a reference plane.

In calculating a translational movement amount of a maxillary bone, a reference plane (X-axis) is set, and then an amount of change in an X-axis direction between a position of a predetermined portion (reference point) on the maxillary bone before movement and a position of the predetermined portion when the maxillary bone is assumed to be moved is calculated as the translational movement amount.

The reference plane can be determined in consideration of reproducibility in the maxillofacial region based on anatomy, and specific examples thereof include an occlusal plane, a Frankfurt plane, and an SN plane.

In addition, the reference point can be appropriately set on the maxillary bone, and a position thereof can be defined based on the measurement point information or the trace line information. Examples of the reference point include ANS, PNS, Point A (the deepest point on the midsagittal section between the anterior nasal spine and the maxillary alveolar ridge), U1, U1R, UMo, and Umo(D).

In addition, movement toward an anterior (labial) side may be assigned a positive sign (+), and movement toward a posterior (lingual) side may be assigned a negative sign (-), thereby distinguishing directions.

Also in calculating a translational movement amount of a mandibular bone (unit: length), a reference plane (X-axis) is set, and then an amount of change in an X-axis direction between a position of a predetermined portion (reference point) on the mandibular bone before movement and a position of the predetermined portion when the mandibular bone is assumed to be moved is calculated as the translational movement amount.

The reference plane can be determined in consideration of reproducibility in the maxillofacial region based on anatomy, and specific examples thereof include an occlusal plane, a Frankfurt plane, and an SN plane.

In addition, the reference point can be appropriately set on the mandibular bone, and a position thereof can be defined based on the measurement point information or the trace line information. Examples of the reference point include Me, Gn (Gnathion, a point at which two lines forming an angle between a facial plane (N-Pog) and a mandibular plane intersect at the mandibular symphysis), Pog, PM (Protuberance menti, an upper margin of a mental protuberance), Point B (the deepest point on the midsagittal section between the anterior border of the mandibular symphysis and the mandibular alveolar ridge), D (D point, a central point of the mandibular symphysis relative to the SN plane), CD (Condylion, the most superoposterior point of the mandibular condyle), CdE (Condyle end, the most posterior point of the mandibular condyle), Ar (Articular, an intersection of a posterior border of the mandibular ramus and an inferior border of the cranial base), Go(P) (Posterior Gonion, a point on a posterior border of the mandibular angle that contacts a posterior border plane of the mandibular ramus), Go, Go(L) (Lower Gonion, a point on a lower border of the mandibular angle that contacts a mandibular plane), Xi (Ricketts point, a center point of the mandibular ramus), a mandibular foramen, and the like.

In addition, movement toward an anterior (labial) side may be assigned a positive sign (+), and movement toward a posterior (lingual) side may be assigned a negative sign (-), thereby distinguishing directions.

An intrusion amount or an extrusion amount of maxillary and mandibular bones (unit: length) is generated as a length between a position of a jaw bone before movement and a position of the jaw bone when assumed to be moved, in a direction perpendicular to a reference plane serving as a reference for movement.

Specifically, an intrusion amount or an extrusion amount of maxillary and mandibular bones (unit: length) can be obtained by calculating a change amount in a length, in a direction perpendicular to the reference plane, between the reference plane and each predetermined portion (reference point) arbitrarily set on the maxillary and mandibular bones.

The reference point can be arbitrarily set and a position thereof can be defined based on the measurement point information or the trace line information. For example, for a maxillary bone, ANS, PNS, Point A, U1, U1R, UMo, Umo(D), and the like can be used; for a mandibular bone, Me, Gn, Pog, PM, Point B, D, CD, CdE, Ar, Go(P), Go, Go(L), Xi, a mandibular foramen, and the like can be used.

The reference plane may be, for example, a Frankfurt plane or an SN plane.

At this time, a case where the maxillary and mandibular bones move toward a cranial side may be expressed as an intrusion amount (for example, with a negative sign (-) assigned with a pre-movement position set as 0), and a case where the maxillary and mandibular bones move toward a caudal side may be expressed as an extrusion amount (for example, with a positive sign (+) assigned with a pre-movement position set as 0).

In the second embodiment, movement amounts of jaw bones can be generated, and movement amounts of anterior teeth and molars that reflect movement of the jaw bones can also be generated.

In FIG. 14, movement amounts of the maxillary and mandibular bones after movement of the maxillary and mandibular bones are calculated with a Frankfurt plane as a reference plane for the maxilla and an SN plane as a reference plane for the mandible, and calculated values representing total movement amounts (a sum of movements before and after surgery) of inclination movement, intrusion/extrusion, and translational movement of the upper and lower anterior teeth and molars accompanying movement of the jaw bones are displayed.

Although the first and second embodiments have been described above, the present invention is not limited thereto and may be implemented in other embodiments.

For example, regarding setting of measurement points, in the first embodiment, after positions of measurement points S and N are input, other measurement points are displayed as provisional positions. However, the present invention is not limited thereto, and an embodiment may be adopted in which the user specifies and inputs all measurement points.

In addition, on a screen displaying movement information, lines indicating planes based on the measurement point information may be displayed superimposed on the standardized head X-ray photograph in addition to the trace lines.

In addition, on a screen displaying movement information, lines indicating bases for calculation of tooth movement information may be displayed superimposed on the standardized head X-ray photograph in addition to the trace lines.

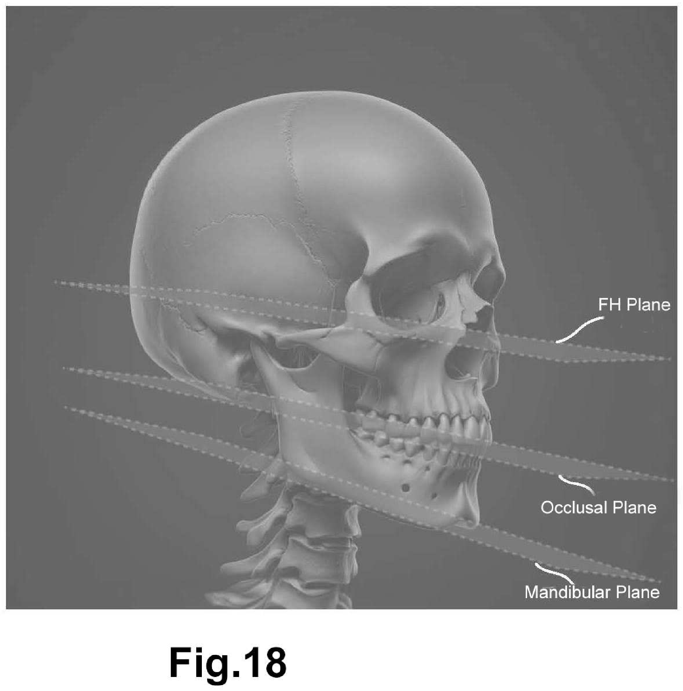

In addition, in the first and second embodiments, a standardized head X-ray photograph is given as an image obtained by imaging with a head X-ray. However, the image is not limited to a standardized head X-ray photograph, and may be, for example, another two-dimensional image or a three-dimensional image obtained by imaging with a head X-ray. As one example, FIG. 18 illustrates a three-dimensional image obtained by imaging with a head X-ray.

In addition, images used in the present invention to obtain measurement point information may be images obtained by a head X-ray image, and may also be images obtained by magnetic resonance imaging (MRI) or computed tomography (CT). Images obtained by MRI or CT may also be either two-dimensional images or three-dimensional images.

In addition, in the first and second embodiments, the treatment support system 100 is configured to include the personal computer 1 and the server S; however, the present invention is not limited thereto, and for example, an embodiment including a plurality of personal computers may be adopted, or an embodiment in which the treatment support system 100 is configured by a single personal computer may be adopted.

### Description of Reference Numerals

1: Personal computer
NW: Network
S: Server
11: Processor
12: Memory
13: SSD
14: Network IF
15: Monitor
16: Input device
17: Media reading device
20: Display unit
22: Operation reception unit
241: Storage unit
242: External storage unit
26: Communication unit
30: Control unit
100: Dental treatment support system

## Claims

1. A dental treatment support system comprising:
a measurement point information acquisition unit configured to acquire measurement point information indicating measurement points corresponding to parts of a head, based on an image obtained by imaging a subject head with an X-ray, a magnetic resonance image of the subject head, or an image obtained by computed tomography of the subject head;
a position information acquisition unit configured to acquire imaging-time position information indicating positions of anterior teeth, molars, or jaw bones of the subject at the time of imaging, based on the measurement point information;
a planned position information acquisition unit configured to acquire planned position information indicating positions in a case where the anterior teeth, molars, or jaw bones are assumed to be moved; and
a movement information generation unit configured to generate movement information indicating amounts of movement from positions of the anterior teeth, molars, or jaw bones before movement to positions in a case where the anterior teeth, molars, or jaw bones are assumed to be moved, based on the measurement point information, the imaging-time position information, and the planned position information.

2. The dental treatment support system according to claim 1,
wherein the movement information includes at least one of:
information indicating an inclination movement amount of anterior teeth or molars,
information indicating an intrusion amount or an extrusion amount of anterior teeth or molars,
information indicating a translational movement amount of anterior teeth or molars,
information indicating an inclination movement amount of jaw bones,
information indicating a translational movement amount of jaw bones, and
information indicating an intrusion amount or an extrusion amount of jaw bones.

3. The dental treatment support system according to claim 2,
wherein the movement information generation unit generates information indicating the inclination movement amount of anterior teeth or molars as an amount of change in an angle of a tooth axis with respect to a reference plane serving as a reference for inclination.

4. The dental treatment support system according to claim 2,
wherein the movement information generation unit generates information indicating the intrusion amount or the extrusion amount of anterior teeth or molars as a length between a position of a tooth before movement and a position of the tooth in a case where the tooth is assumed to be moved, in a direction perpendicular to a reference plane serving as a reference for movement.

5. The dental treatment support system according to claim 2,
wherein the movement information generation unit generates information indicating the translational movement amount of anterior teeth or molars as a length between a position of a tooth before movement and a position of the tooth in a case where the tooth is assumed to be moved, in a direction along a reference plane serving as a reference for movement.

6. The dental treatment support system according to claim 2,
wherein the movement information generation unit generates information indicating the inclination movement amount of jaw bones as an amount of change in an angle between a reference plane serving as a reference for inclination and a plane that is at least in contact with a jaw bone.

7. The dental treatment support system according to claim 2,
wherein the movement information generation unit generates information indicating the intrusion amount or the extrusion amount of jaw bones as a length between a position of a jaw bone before movement and a position of the jaw bone in a case where the jaw bone is assumed to be moved, in a direction perpendicular to a reference plane serving as a reference for movement.

8. The dental treatment support system according to claim 2,
wherein the movement information generation unit generates information indicating the translational movement amount of jaw bones as a length between a position of a jaw bone before movement and a position of the jaw bone in a case where the jaw bone is assumed to be moved, in a direction along a reference plane serving as a reference for movement.

9. The dental treatment support system according to any one of claims 1 to 8, further comprising:
a position identification support unit configured to generate position information generation support information for supporting identification of positions of anterior teeth, molars, or jaw bones of the subject using the measurement point information acquired by the measurement point information acquisition unit,
wherein the position information acquisition unit acquires the imaging-time position information based on user input using the position information generation support information generated by the position identification support unit.

10. The dental treatment support system according to any one of claims 1 to 8,
wherein the measurement point information acquisition unit acquires the measurement point information based on a standardized head X-ray photograph that is an image obtained by imaging the head with an X-ray.

11. An information processing method executed by a computer, comprising:
acquiring measurement point information indicating measurement points corresponding to parts of a head, based on an image obtained by imaging a subject head with an X-ray, a magnetic resonance image of the subject head, or an image obtained by computed tomography of the subject head;
acquiring imaging-time position information indicating positions of anterior teeth, molars, or jaw bones of the subject at the time of imaging, based on the measurement point information;
acquiring planned position information indicating positions in a case where the anterior teeth, molars, or jaw bones are assumed to be moved; and
generating movement information indicating amounts of movement from positions of the anterior teeth, molars, or jaw bones before movement to positions in a case where the anterior teeth, molars, or jaw bones are assumed to be moved, based on the measurement point information, the imaging-time position information, and the planned position information.

12. The method according to claim 11,
wherein the movement information includes at least one of:
information indicating an inclination movement amount of anterior teeth or molars,
information indicating an intrusion amount or an extrusion amount of anterior teeth or molars,
information indicating a translational movement amount of anterior teeth or molars,
information indicating an inclination movement amount of jaw bones,
information indicating a translational movement amount of jaw bones, and
information indicating an intrusion amount or an extrusion amount of jaw bones.

13. The method according to claim 12,
wherein information indicating the inclination movement amount of anterior teeth or molars is generated as an amount of change in an angle of a tooth axis with respect to a reference plane serving as a reference for inclination.

14. The method according to claim 12,
wherein information indicating the intrusion amount or the extrusion amount of anterior teeth or molars is generated as a length between a position of a tooth before movement and a position of the tooth in a case where the tooth is assumed to be moved, in a direction perpendicular to a reference plane serving as a reference for movement.

15. The method according to claim 12,
wherein information indicating the translational movement amount of anterior teeth or molars is generated as a length between a position of a tooth before movement and a position of the tooth in a case where the tooth is assumed to be moved, in a direction along a reference plane serving as a reference for movement.

16. The method according to claim 12,
wherein information indicating the inclination movement amount of jaw bones is generated as an amount of change in an angle between a reference plane serving as a reference for inclination and a plane that is at least in contact with a jaw bone.

17. The method according to claim 12,
wherein information indicating the intrusion amount or the extrusion amount of jaw bones is generated as a length between a position of a jaw bone before movement and a position of the jaw bone in a case where the jaw bone is assumed to be moved, in a direction perpendicular to a reference plane serving as a reference for movement.

18. The method according to claim 12,
wherein information indicating the translational movement amount of jaw bones is generated as a length between a position of a jaw bone before movement and a position of the jaw bone in a case where the jaw bone is assumed to be moved, in a direction along a reference plane serving as a reference for movement.

19. The method according to any one of claims 11 to 18, further comprising:
generating position information generation support information for supporting identification of positions of anterior teeth, molars, or jaw bones of the subject using the measurement point information,
wherein the imaging-time position information is acquired based on user input using the position information generation support information.

20. The method according to any one of claims 11 to 18,
wherein the measurement point information is acquired based on a standardized head X-ray photograph that is an image obtained by imaging the head with an X-ray.

21. A program for causing a computer to function as:
a measurement point information acquisition unit configured to acquire measurement point information indicating measurement points corresponding to parts of a head, based on an image obtained by imaging a subject head with an X-ray, a magnetic resonance image of the subject head, or an image obtained by computed tomography of the subject head;
a position information acquisition unit configured to acquire imaging-time position information indicating positions of anterior teeth, molars, or jaw bones of the subject at the time of imaging, based on the measurement point information;
a planned position information acquisition unit configured to acquire planned position information indicating positions in a case where the anterior teeth, molars, or jaw bones are assumed to be moved; and
a movement information generation unit configured to generate movement information indicating amounts of movement from positions of the anterior teeth, molars, or jaw bones before movement to positions in a case where the anterior teeth, molars, or jaw bones are assumed to be moved, based on the measurement point information, the imaging-time position information, and the planned position information.

22. The program according to claim 21,
wherein the movement information includes at least one of:
information indicating an inclination movement amount of anterior teeth or molars,
information indicating an intrusion amount or an extrusion amount of anterior teeth or molars,
information indicating a translational movement amount of anterior teeth or molars,
information indicating an inclination movement amount of jaw bones,
information indicating a translational movement amount of jaw bones, and
information indicating an intrusion amount or an extrusion amount of jaw bones.

23. The program according to claim 22,
wherein the movement information generation unit generates information indicating the inclination movement amount of anterior teeth or molars as an amount of change in an angle of a tooth axis with respect to a reference plane serving as a reference for inclination.

24. The program according to claim 22,
wherein the movement information generation unit generates information indicating the intrusion amount or the extrusion amount of anterior teeth or molars as a length between a position of a tooth before movement and a position of the tooth in a case where the tooth is assumed to be moved, in a direction perpendicular to a reference plane serving as a reference for movement.

25. The program according to claim 22,
wherein the movement information generation unit generates information indicating the translational movement amount of anterior teeth or molars as a length between a position of a tooth before movement and a position of the tooth in a case where the tooth is assumed to be moved, in a direction along a reference plane serving as a reference for movement.

26. The program according to claim 22,
wherein the movement information generation unit generates information indicating the inclination movement amount of jaw bones as an amount of change in an angle between a reference plane serving as a reference for inclination and a plane that is at least in contact with a jaw bone.

27. The program according to claim 22,
wherein the movement information generation unit generates information indicating the intrusion amount or the extrusion amount of jaw bones as a length between a position of a jaw bone before movement and a position of the jaw bone in a case where the jaw bone is assumed to be moved, in a direction perpendicular to a reference plane serving as a reference for movement.

28. The program according to claim 22,
wherein the movement information generation unit generates information indicating the translational movement amount of jaw bones as a length between a position of a jaw bone before movement and a position of the jaw bone in a case where the jaw bone is assumed to be moved, in a direction along a reference plane serving as a reference for movement.

29. The program according to any one of claims 21 to 28,
wherein the computer is further caused to function as a position identification support unit configured to generate position information generation support information for supporting identification of positions of anterior teeth, molars, or jaw bones of the subject using the measurement point information acquired by the measurement point information acquisition unit,
and the position information acquisition unit acquires the imaging-time position information based on user input using the position information generation support information generated by the position identification support unit.

30. The program according to any one of claims 21 to 28,
wherein the measurement point information acquisition unit acquires the measurement point information based on a standardized head X-ray photograph that is an image obtained by imaging the head with an X-ray.
